# EUROPEAN PATENT APPLICATION

(11) **EP 4 187 311 A1**
(43) Date of publication of application: **31.05.2023**
(21) Application number: 21306647.5
(22) Date of filing: 26.11.2021
(51) Int. Cl.: G02C 7/00, G16H 50/50, G16H 50/70

(54) **COMPUTER-IMPLEMENTED METHOD, APPARATUS, SYSTEM AND COMPUTER PROGRAM FOR PROVIDING A USER WITH A REPRESENTATION OF AN EFFECT OF A SIGHTEDNESS IMPAIRMENT CONTROL SOLUTION**

(71) Applicant: Essilor International, 94220 Charenton-Le-Pont (FR)
(72) Inventor: PERRIN, Jean-Luc, Shanghai, 200000 (CN)
(74) Representative: Korakis-Ménager, Sophie

(57) **Abstract**

Computer-implemented method, apparatus, system and computer program, for providing a user with a representation of an effect of a sightedness impairment control solution on the user, the computer-implemented method comprising the steps of obtaining data representative of at least one characteristic of the sightedness impairment control solution, at least one current characteristic of the user and behaviour of the user, determining, based on a model and the data, at least one first future characteristic of the user if the user uses the sightedness impairment control solution and at least one second future characteristic of the user if the user does not use the sightedness impairment control solution, and generating a first image based on the at least one first future characteristic and a second image based on the at least one second future characteristic.

## Description

### FIELD

Various aspects of this disclosure generally relate to sightedness impairment control solution for example myopia control solution and more particularly, to methods and apparatus for providing a user with a representation of an effect of a sightedness impairment control solution.

### BACKGROUND

Sightedness impairment, for example myopia, is a condition that is often evolving in a progressing manner, furthermore, it concerns more and more children. Myopia progression and high myopia can lead to consequences affecting the future quality of life of the child (vision performance, vision quality, appearance, visual health...).

Many type of products for slowing down myopia progression can be used, such as ophthalmic lenses, contact lenses or drugs. For example, to avoid a myopia progression that is due to such focusing defect, it is known to use a myopia-correcting lens that is of the progressive multifocal ophthalmic lens type. Bifocal lenses may also be an example of ophthalmic lenses that can be used to slow down myopia progression.

The eye care professional (ECP) needs to educate the user or the user's relatives of the interest of sightedness impairment control solutions before the patient can benefit from such a solution. The relatives are any person influencing or defining the choice of the sightedness impairment control solutions, for example the parents of the user, members of the family of the user or legal guardian of the user. But the user or the user's parents can have poor awareness about sightedness impairment, dynamic of the sightedness impairment progression and sightedness impairment control solutions. They can only rely on the explanations of the ECP, who can be perceived as having commercial interest to dispense this type of solutions and can lack objective information to make a decision.

Therefore, there is a need for a solution to let the user experience the future benefit of a sightedness impairment control solution, while he/she cannot experience it today.

### SUMMARY

The following presents a simplified summary in order to provide a basic understanding of various aspects of this disclosure. This summary is not an extensive overview of all contemplated aspects, and is intended to neither identify key or critical elements of all aspects nor delineate the scope of any or all aspects. The sole purpose is to present some concepts of one or more aspects in a simplified form as a prelude to the more detailed description that is presented later.

One aspect of this disclosure is a computer-implemented method for providing a user with a representation of an effect of a sightedness impairment control solution on the user, the computer-implemented method comprising the steps of obtaining data representative of at least one characteristic of the sightedness impairment control solution, at least one current characteristic of the user and behaviour of the user, and determining, based on a model and the data, at least one first future characteristic of the user if the user uses the sightedness impairment control solution and at least one second future characteristic of the user if the user does not use the sightedness impairment control solution, generating a first image based on the at least one first future characteristic and a second image based on the at least one second future characteristic.

Another aspect of this disclosure is an apparatus for providing a user with a representation of an effect of a sightedness impairment control solution on the user, the apparatus comprising a memory and at least one processor coupled to the memory. The processor is configured to obtain data representative of at least one characteristic of the sightedness impairment control solution, at least one current characteristic of the user and behaviour of the user, determine, based on a model and the data, at least one first future characteristic of the user will need if the user uses the sightedness impairment control solution and at least one second future characteristic of the user will need if the user does not use the sightedness impairment control solution, and generate a first image based on the at least one first future characteristic and a second image based on the at least one second future characteristic.

In an embodiment, the apparatus also comprises a display configured to display the first image and/or the second image and/or, a camera configured to capture an image of a face of the user.

Another aspect of this disclosure is a system comprising, the apparatus and a sightedness impairment control solution.

In an embodiment of the system, the sightedness impairment control solution comprises at least one ophthalmic lens.

In an embodiment of the system, the at least one ophthalmic lens is characterized by an optical power in at least one location, the at least one location being for example a near vision point or a far vision point.

In an embodiment of the system, the sightedness impairment control solution is a myopia control solution and the myopia control solution comprises at least a device for implementing one of the following actions or several of the following actions simultaneously:
- correcting or reducing an accommodative lag during near vision activities;
- correcting peripheral hyperopic defocus or providing myopic defocus by;
- providing retinal stimulation;
- providing a different contrast in a peripheral vision of the user;
- limiting the amount of red light entering an eye of the user to reduce chromatism of the eye;
- providing light having a specific wavelength to the eye to inhibit eye lengthening ;
- providing dynamically varying light stimuli such as flickering;
- providing drugs to regulate retinal and scleral muscarinic receptors ;
- reshaping a cornea of the eye to reduce refraction defects ;
- providing myopic defocus in the retinal periphery and/or optical aberrations by flattening the shape of the cornea.

Another aspect of this disclosure is a computer program for providing a user with a representation of an effect of a sightedness impairment control solution on the user, the computer program comprising instructions which, when the computer program is executed by a computer, cause the computer to carry out steps of obtaining data representative of at least one characteristic of the sightedness impairment control solution, at least one current characteristic of the user and behaviour of the user, determining, based on a model and the data, at least one first future characteristic of the user if the user uses the sightedness impairment control solution and at least one second future characteristic of the user if the user does not use the sightedness impairment control solution, and generating a first image based on the at least one first future characteristic and a second image based on the at least one second future characteristic.

### DESCRIPTION OF THE DRAWINGS

For a more complete understanding of the description provided herein and the advantages thereof, reference is now made to the brief descriptions below, taken in connection with the accompanying drawings and detailed description, wherein like reference numerals represent like parts.
Figure 1 is a schematic representation of the system for providing a user with a representation of an effect of a sightedness impairment control solution.
Figure 2 is a schematic representation of an apparatus for providing a user with a representation of an effect of a sightedness impairment control solution.
Figure 3 is a schematic representation of a method for providing a user with a representation of an effect of a sightedness impairment control solution.
Figure 4 is an illustration of data contained within a data bank of patients using or not a sightedness impairment control solution.
Figure 5 is another schematic representation of a method for providing a user with a representation of an effect of a sightedness impairment control solution.
Figures 6-a and 6-b are images representing the effect of the sightedness impairment control solution.
Figures 7-a and 7-b are images of a lateral view of lenses.
Figures 8-a and 8-b are images of a lateral view of the eye of the user.

### DETAILED DESCRIPTION OF EMBODIMENTS

The detailed description set forth below in connection with the appended drawings is intended as a description of various possible embodiments and is not intended to represent the only embodiments in which the concepts described herein may be practiced. The detailed description includes specific details for the purpose of providing a thorough understanding of various concepts. However, it will be apparent to those skilled in the art that these concepts may be practiced without these specific details. In some instances, well-known structures and components are shown in block diagram form in order to avoid obscuring such concepts.

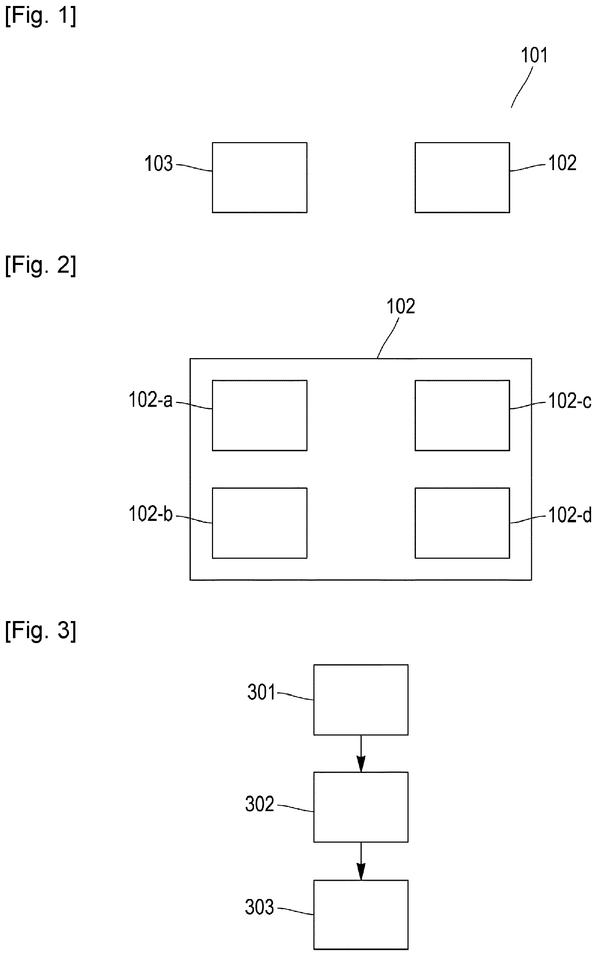

One of the embodiments, described in figure 1, concerns a system 101 for providing a user with a representation of an effect of a sightedness impairment control solution 103. This system comprises an apparatus 102 and the sightedness impairment control solution 103. The apparatus is configured to provide the user with a representation of an effect of the sightedness impairment control solution.

The figure 2 represents the apparatus 102. This apparatus 102 comprises a memory 102-a and a processor 102-b coupled to the memory 102-a. This apparatus can also comprise a display 102-c and a camera 102-d. This camera 102-d can be for example configured to capture an image of a face of the user.

Examples of processors include microprocessors, microcontrollers, graphics processing units (GPUs), central processing units (CPUs), application processors, digital signal processors (DSPs), reduced instruction set computing (RISC) processors, systems on a chip (SoC), baseband processors, field programmable gate arrays (FPGAs), programmable logic devices (PLDs), state machines, gated logic, discrete hardware circuits, and other suitable hardware configured to perform the various functionality described throughout this disclosure.

The memory 102-a is computer-readable media. By way of example, and not limitation, such computer-readable media may include a random-access memory (RAM), a read-only memory (ROM), an electrically erasable programmable ROM (EEPROM), optical disk storage, magnetic disk storage, other magnetic storage devices, combinations of the aforementioned types of computer-readable media, or any other medium that can be used to store computer executable code in the form of instructions or data structures that can be accessed by a computer.

The memory 102-a is configured to store a computer program comprising instructions which, when the program is executed by the processor 102-b, cause the apparatus 102 to carry out the steps of a method for providing a user with a representation of an effect of a sightedness impairment control solution.

Figure 3 represents the method for providing a user with a representation of an effect of a sightedness impairment control solution.

The method of figure 3 comprises
- a step 301 of obtaining data representative of a characteristic of the sightedness impairment control solution 103, a current characteristic of the user and behaviour of the user, and
- a step 302 of determining, based on a model and the data, a first future characteristic of the user if the user uses the sightedness impairment control solution 103 and a second future characteristic of the user if the user does not use the sightedness impairment control solution 103,
- a step 304 of generating a first image based on the first future characteristic and a second image based on the second future characteristic.

The user does not use the sightedness impairment control solution 103 means that, the user does not use any sightedness impairment control solution 103 or the user uses a different type of sightedness impairment control solution 103.

The system 101 allows for one given user, based on his/her data, to provide an estimate of the evolution of sightedness impairment (with a standard correction, no sightedness impairment control). The system 101 also allows to provide an estimate of the evolution of sightedness impairment of the user with a sightedness impairment control solution and to display this estimation in an intelligible way.

This system 101 allows the user or a relative of the user to easily understand the impact of the solution's choice on sightedness impairment development, they make an informed choice.

The model can, for example, comprise a plurality of cases. Each case is associated with a patient, an indication of an usage of the sightedness impairment control solution by the patient, at least one characteristic of the patient, behaviour of the patient and an evolution of a sightedness impairment of the patient.

The first future characteristic of the user and the second future characteristic of the user can be related to a myopia of the user. More precisely they can represent a level of myopia.

The level of myopia can be expressed in different ways:
- Spherical error: monocular and/or binocular Spherical Equivalent, refraction or prescription
- Axial length: left eye, right eye, average of both eyes. Complemented or not with corneal parameters (K1, K2)
- Presence or absence of myopia (at a given age)

The step 302 of determining the first future characteristic of the user and the second future characteristic of the user are realized based on:
- Any optometric parameter (visual acuity, binocular measurements,...)
- Any behavioural parameter (time spent outside, time spent using near vision,...)
- Any genetic parameter (number of parents with myopia, siblings with myopia...)

In a nutshell, the current characteristic of the user and/or the patient comprises some or a part of the following characteristics:
- a spherical equivalent of an eye and an age.
- a spherical error of a right eye and/or a left eye of the user and/or
- a monocular and/or binocular spherical equivalent of the right eye and/or the left eye and/or
- a refraction or a prescription on the right eye and/or the left eye and/or
- an axial length of the left eye and/or the right eye or an average of the axial length of the left eye and the axial length of the right eye and/or
- at least one corneal parameter of the left eye and/or the right eye and/or
- an indication of a presence of myopia on the left eye and/or the right eye and/or
- a variation of any aforementioned characteristics for a given period of time.

The behaviour of the user comprises proportions of time spent in near and far vision.

In an embodiment, the step 302 of determining the first future characteristic and the second future characteristic comprises:
- A step of selecting, among the cases, a first case associated with a patient not using the sightedness impairment control solution 103 and with the characteristic and/or the behaviour of the patient the closest to the characteristic and/or the behaviour of the user,
- A step of selecting, among the cases, a second case associated with a patient using the sightedness impairment control solution 103 and with the characteristic and/or the behaviour of the patient the closest to the characteristic and/or the behaviour of the user,
- A step of determining the first future characteristic based on a current sightedness impairment of the user and the evolution of the sightedness impairment of the patient of the first case,
- A step of determining the second future characteristic based on the current sightedness impairment of the user and the evolution of the sightedness impairment of the patient of the second case.

In other words, in this embodiment, the model is a general data bank of existing cases, the closest case compared to the current user is extracted and serves as an estimation (cases available within an internal database, scientific publications, etc). The bank can also be formed by the family members (who can have similar genetics and behavioural factors), then the patient is compared to a sibling.

The bank can advantageously be updated constantly with new data sets of cases, so that as the sampling set gets larger, the easier to identify one or several myopia "doppelganger" with a profile the most similar (age, ethnicity, geographical zone of residence, similar family myopia history, ...) to the user to make the prediction. Once one or several myopia "doppelganger" is identified, then the predictions on the efficiency of myopia control solution will be based on his doppelganger. Optionally, concrete recommendations to improve of lifestyle could be made based on the data of one's myopia "doppelgänger" for example increase wearing time of the solution, adopt longer reading distance when having a near work activity.

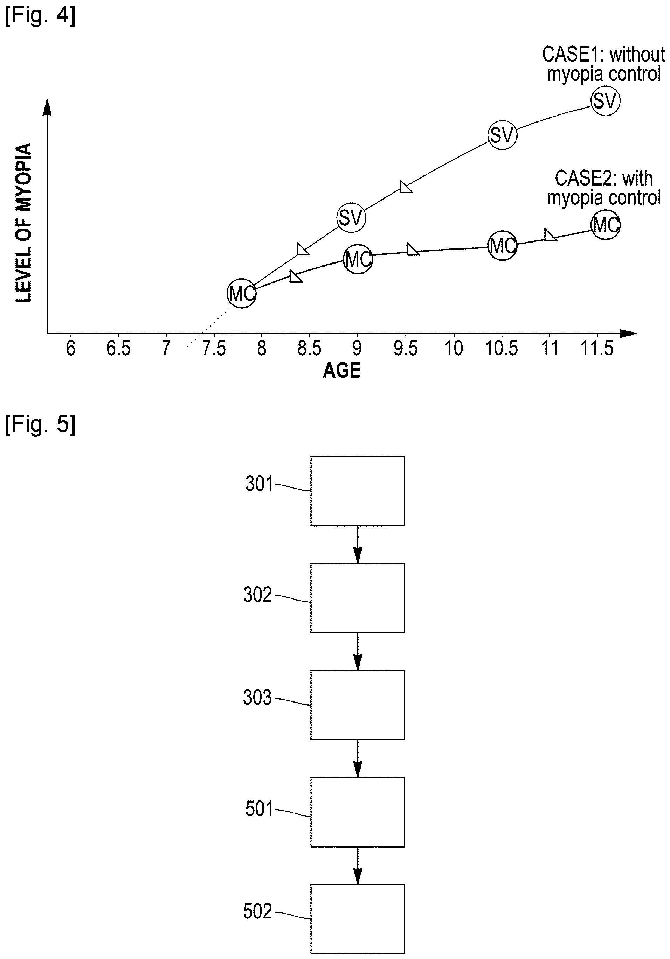

The figure 4 is an illustration of this data contained in the data bank.

Typically, this data bank is filled during a clinical trial involving a control group of patients and a test group of patients.

The patients of the control group are not using the myopia correction solution, whereas the patients of the test group are using the myopia control solution.

For example, patients of the control group used classical ophthalmic single vision lenses, whereas the patients of the test group used the myopia control solution.

The evolution of the myopia is followed for the patients of each of the control group and test group by determining at different times during the clinical trial the value of a magnitude representative of the myopia degree of the eyes of each patient of the control group and test group. The axial length of the eye or the axial elongation (difference between current value of the axial length and a reference value) are examples of such magnitude representative of the myopia degree of the eyes. The spherical equivalent refraction or the absolute value of the spherical equivalent refraction of the eye is another example. Said magnitude representative of the myopia degree of the eyes may also, for example, be determined based on the power of the ideal dioptric correction needed by the eye, measured in diopters.

This embodiment can be implemented the following way:
For a given user (User A), with a specific age at the time of the visit (age_A) and a specific spherical equivalent (SE) correction value for each of his/her eyes (seR_A, seL_A). The selection criteria could be the following:
Within the database of existing cases, the algorithm will return all the cases (a case include all the datapoints: spherical equivalent + age from each visit)

According that the case x includes at least one point:
- seR_x is within the range [seR_A - 0.25 ; seR_A + 0.25] (unit is diopters) and
- age_x is within the range [age_A - 0.5 ; age_A + 0.5] (unit is years)

The search of similar case can be based on any or both of the eyes (seR_A and seL_A)

The points considered for the case x can be the coordinates (age, spherical equivalent) for each visit of the given patient. In order to increase the number of cases found, one can use an interpolation. In this case, the spherical equivalent of user x can be provided as the result of the function: seR_x = f(age_x). The comparison of the individual points is therefore realized on the curve of the previous function.

Alternatively, the comparison can be performed on several points. If we have two consecutive points for user A: (age_1A, seR_1A) and (age_2A, seR_2A). We can compute a myopia progression speed using equation, speed_A = (seR_2A - seR_1A) / (age_2A - age_1A) [unit: diopters per year]. In this case, the steps of selecting would thus identify patients with similar progression speed (speed_A +/- 0.125 diopters per years) at a similar age (age_2A +/- 0.5 years). This comparison can be performed for one of the eyes or the other, or both. This comparison can also be performed on an interpolation (function), in this case the comparison is made with the curve slope.

In an embodiment, to conserve single cases confidentiality, the algorithm can return average values of the selected cases.

In an embodiment, the step 302 of determining the first future characteristic of the user and the second future characteristic of the user is realized based on references to a percentile model: given the description of the level of myopia of a representative population for different time periods, the user is situated within the population. Using the nearest percentile allows to predict the evolution of myopia. Such models can be derived from epidemiological studies (published in scientific literature, or unpublished, based on data collection). Such percentile model is currently commonly used in different domains of healthcare (child growth height/ weight/ etc...).

In an embodiment, the step 302 of determining the first future characteristic of the user and the second future characteristic of the user is realized using a personalized myopia prediction model. This personalized myopia prediction model can be obtained using the method described in the PCT application reference WO2011000777. A computer-based model derives from the input parameters a prediction of the future myopia level(s). The model can be theoretical or empirical for example based on machine learning, trained with real population data.

In an embodiment, the step 302 of determining the first future characteristic of the user and the second future characteristic of the user is performed by the ECP himself, based on experience - he/she inputs the level of myopia he/she is used to observe for similar patients

In an embodiment, the step 302 of determining the first future characteristic of the user and the second future characteristic of the user is realized by training a neural network, by using a plurality of cases, at least one case being associated with a patient, an indication of a usage of the sightedness impairment control solution 103 by the patient, at least one characteristic of the patient, behaviour of the patient and an evolution of a sightedness impairment of the patient.

In other words, in this embodiment, the model is based on machine learning, the training dataset can include patients using the sightedness impairment control solution 103. Thus the step 302 can predict a level of sightedness impairment with the sightedness impairment control solution 103 and a level without the sightedness impairment control solution 103.

In an embodiment, the sightedness impairment control solution 103 comprises one ophthalmic lens and the at least one characteristic of the sightedness impairment control solution 103 is an optical characteristic. This sightedness impairment control solution 103 can be a myopia control solution.

In an embodiment, the optical characteristics of the sightedness impairment control solution 103 comprise an optical power at a location of an ophthalmic lens.

In this embodiment, the one location comprises at least one of a near vision point or a far vision point of the ophthalmic lens.

In an embodiment, the sightedness impairment control solution 103 is a myopia control solution and the myopia control solution comprises at least a device for implementing one of the following actions or several of the following actions simultaneously:
- correcting or reducing an accommodative lag during near vision activities;
- correcting peripheral hyperopic defocus or providing myopic defocus by;
- providing retinal stimulation;
- providing a different contrast in a peripheral vision of the user;
- limiting the amount of red light entering an eye of the user to reduce chromatism of the eye;
- providing light having a specific wavelength to the eye to inhibit eye lengthening ;
- providing dynamically varying light stimuli such as flickering;
- providing drugs to regulate retinal and scleral muscarinic receptors ;
- reshaping a cornea of the eye to reduce refraction defects ;
- providing myopic defocus in the retinal periphery and/or optical aberrations by flattening the shape of the cornea.

Other types of myopia control solutions may be used. These myopia control solutions have been described and tested and are currently used to limit the progression of myopia in subjects.

The accommodative lag of the eye is defined as the difference between accommodative demand and accommodative response and resulting in a residual refractive error.

Each myopia control solution may comprise one or several basic myopia control solution as described above. In practice, these actions are performed with five types of devices for controlling myopia:
- Ophthalmic lenses integrated to spectacles,
- Contact lenses worn directly on the eye of the subject,
- Light sources, for example to reproduce daily light indoors or avoid emitting wavelengths responsible for eye elongation,
- Filters such as blue-cut filters cutting wavelengths responsible for eye elongation, for example integrated to a lens or comprising digital screen filters,
- Pharmaceutical products.

Each myopia control solution may perform one or several actions listed above. Each myopia control solution may use one or several devices for controlling myopia.

For example, correcting or reducing the accommodative lag during near vision activities is done using a lens having a positive sphere power in the region of the lens used in near vision tasks. It may be a bifocal lens comprising a zone with a positive sphere located in the near vision region of the lens or a progressive lens with addition in the near vision region. An example of bifocal lenses used to control myopia may be a prismatic bifocal lens having two optical zones: the zone located in the far vision region (upper part of the lens) compensate the myopic refraction defect of the eye, whereas the zone located in the near vision region (lower part of the lens) reduces the accommodation demand to see at near end and therefore the accommodative lag.

Correcting peripheral hyperopic defocus or providing myopic defocus may be achieved by using a lens having a positive sphere power in the periphery of the lens.

Customized lenses providing retinal stimulation using multiple light stimuli located in front of retina of the eye of the subject may be used, which comprises:
- a refraction area having a first refractive power based on a prescription for correcting an abnormal refraction of said eye; and
- a plurality of at least three optical elements, at least one optical element having an optical function of not focusing an image on the retina of the eye so as to slow down the progression of the abnormal refraction of the eye;

Such lenses are described for instance in the PCT application WO2019166654.

Providing a different contrast in peripheral vision may be achieved by inserting scattering elements in the periphery of the lens.

Limiting the amount of red light entering the eye may be achieved using a specific filter, for example integrated to a lens.

Limiting the amount of red light entering the eye may be achieved using a specific filter, for example integrated to a lens.

Providing light having a specific wavelength to the eye to inhibit eye lengthening may be achieved by luminotherapy involving exposing eyes to the wavelength that triggers the dopamine receptors regulating eye elongation.

Providing dynamically varying light stimuli may be achieved by exposing eyes to sinusoidal modulation of white light (with blue or with yellow) according to a temporal frequency.

Providing drugs to regulate retinal and scleral muscarinic receptors may be achieved for example by providing topical atropine directly delivered in the eye of the subject using eye drops. Other pharmacological approaches trialed for myopia control include topical timolol, a nonselective beta-adrenergic antagonist, and oral 7-methylxanthine (7-MX), an adenosine antagonist. The effect of atropine for myopia control has been shown is several studies, such as the study of Wu, P.-C., Chuang, M.-N., Choi, J., Chen, H., Wu, G., Ohno-Matsui, K., Jonas, J.B., Cheung, C.M.G., 2019, « Update in myopia and treatment strategy of atropine use in myopia control » in Eye 33, 3-13.

The effect of the drugs that may be provided to the eye typically lasts for at least a few hours. This is why when implemented with another myopia control solution the use of the drugs is considered simultaneous.

Corneal reshaping, also known as orthokeratology, is performed by fitting a specially designed gas permeable contact lens on the eye of the subject. The contact lens is typically flattened and reshapes the cornea of the eye to reduce the myopic refraction defect.

Providing myopic defocus in the retinal periphery and/or optical aberrations by flattening the shape of the cornea may be achieved for example by using an ortho-K solution.

Any other myopia control solution known for the man skilled in the art may be taken into account. All possible combinations of basic myopia control solutions may be taken into account, and therefore included in said database 101 in said memory 100. Usually, combinations of two basic myopia control solutions are considered, such as the combination of the use of pharmaceutical products such as atropine in combination with any other myopia control solution. The combination lenses providing retinal stimulation with the use of a positive sphere zone in the near vision region could also be considered.

The efficacy of said myopia control solution is defined as a magnitude representative of the ability of this myopia control solution to produce a desired or intended result in controlling the myopia of the wearer. The efficacy may for example be quantified as a percentage value, 100% being the highest efficacy and 0% being the lowest efficacy.

In an embodiment presented figure 5 the method for providing a user with a representation of an effect of a sightedness impairment control solution. In this embodiment, the method comprises:
- a step of determining 501 a first optical article based on the at least one first future characteristic of the user and,
- a step of determining 502 a second optical article based on the at least one second future characteristic of the user.

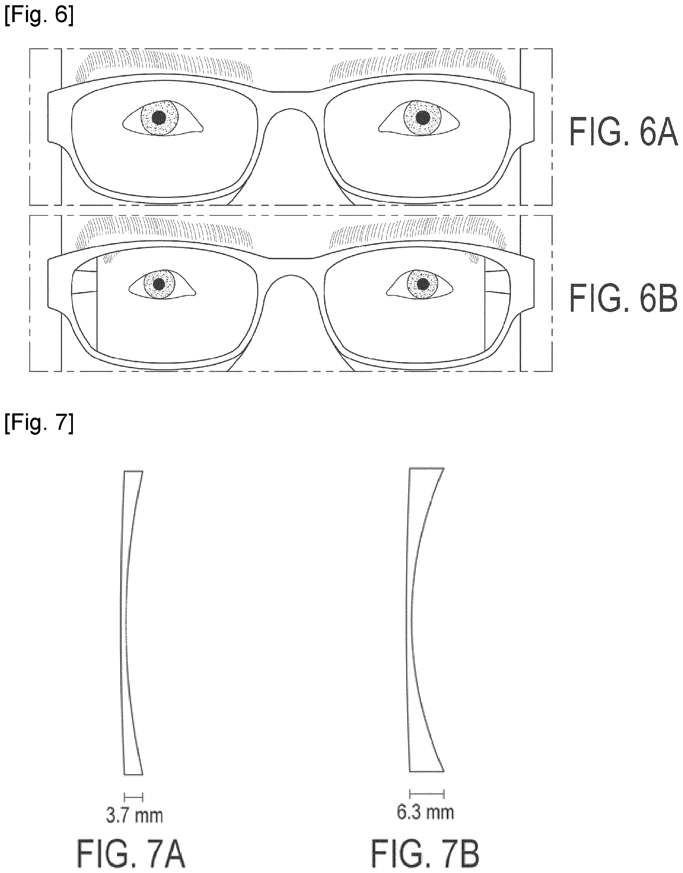

In an embodiment, the first image is an image of a face of the user wearing the first optical article and the second image is an image of the face of the user wearing the second optical article. The figure 6-a and 6-b are examples of these two images. Figure 6-a is a representation of the face of the user if he wears the first optical article and figure 6-b is a representation of the face of the user if he wears the second optical article. The first optical article has an optical power inferior to the one of the second optical article because the user, in the first case, wore the sightedness impairment control solution 103, and then the sightedness impairment is smaller. Because of this reduction of optical power, the temporal jump and the reduction of the eye as saw through the lenses are smaller in figure 6-a than in figure 6-b. The temporal jump is the optical effect due to the wearing of myopic lenses, seen by an observer in front of the wearer, resulting in a lateral shift toward the nose of the wearer's temples. The myopic rings are also smaller in figure 6-a than in figure 6-b. The myopic rings are the optical effect due to the wearing of myopic lenses, seen by an observer situated beside the wearer, resulting in white circles at the edge of the lens. The European patent application reference EP2629140B1 describes an embodiment of the generation of the image of the face of the user wearing the first optical article and the image of the face of the user wearing the first optical article.

In another embodiment, the first image is an image of a shape of the first lens and the second image is an image of a shape of the second lens. Figures 7-a and 7-b are examples of these two images.

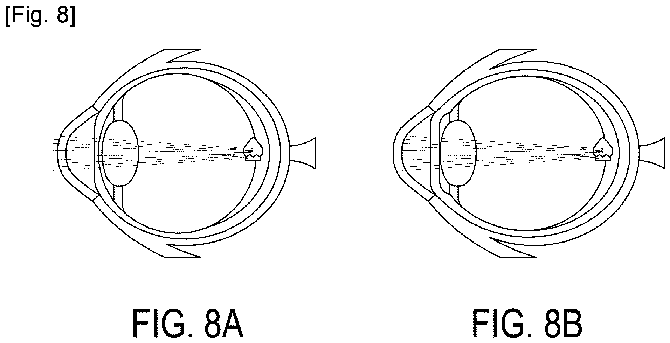

In an embodiment, the first image and the second image are images of a lateral view (for example a two dimensions or 2D lateral view) of the eye. Generally, the first image will be a more myopic eye than the second image. Figures 8-a and 8-b are examples of these two images.

In an embodiment, the first image and the second image are a table comprising the risks of ocular disease associated with the first and second future characteristics of the user. These risks are described in the article "IMI Impact of Myopia", more precisely the table 3 of this article. This article was published in Investigative Ophthalmology & Visual Science April 2021, Vol.62, 2 and also accessible from the following web page: https://iovs.arvojournals.org/article.aspx?articleid=2772540.

## Claims

1. Computer-implemented method for providing a user with a representation of an effect of a sightedness impairment control solution (103) on the user, the computer-implemented method comprising the steps of:
- obtaining (301) data representative of at least one characteristic of the sightedness impairment control solution, at least one current characteristic of the user and behaviour of the user,
- determining (302), based on a model and the data, at least one first future characteristic of the user if the user uses the sightedness impairment control solution (103) and at least one second future characteristic of the user if the user does not use the sightedness impairment control solution (103), and
- generating (303) a first image based on the at least one first future characteristic and a second image based on the at least one second future characteristic.

2. The computer-implemented method according to claim 1 wherein the model comprises a plurality of cases, at least one case being associated with a patient, an indication of a usage of the sightedness impairment control solution (103) by the patient, at least one characteristic of the patient, behaviour of the patient and an evolution of a sightedness impairment of the patient,
the step of determining the at least one first future characteristic and the at least one second future characteristic comprising:
- select, among the cases, a first case associated with a patient not using the sightedness impairment control solution (103) and with the at least one characteristic and/or the behaviour of the patient the closest to the at least one characteristic and/or the behaviour of the user,
- select, among the cases, a second case associated with a patient using the sightedness impairment control solution (103) and with the at least one characteristic and/or the behaviour of the patient the closest to the at least one characteristic and/or the behaviour of the user,
- determine the at least one first future characteristic based on a current sightedness impairment of the user and the evolution of the sightedness impairment of the patient of the first case,
- determine the at least one second future characteristic based on the current sightedness impairment of the user and the evolution of the sightedness impairment of the patient of the second case.

3. The computer-implemented method according to claim 1 wherein the model is a neural network and the computer-implemented method also comprises: training the neural network, by using a plurality of cases, at least one case being associated with a patient, an indication of a usage of the sightedness impairment control solution (103) by the patient, at least one characteristic of the patient, behaviour of the patient and an evolution of a sightedness impairment of the patient.

4. The computer-implemented method according to any one of the claims 1 to 3 wherein the at least one current characteristic of the user and/or the patient comprises a spherical equivalent of an eye and/or an age.

5. The computer-implemented method according to any one of the claims 1 to 4 wherein the at least one characteristic of the user and/or the at least one future characteristic of the user comprises :
- a spherical error of a right eye and/or a left eye of the user and/or
- a monocular and/or binocular spherical equivalent of the right eye and/or the left eye and/or
- a refraction or a prescription on the right eye and/or the left eye and/or
- an axial length of the left eye and/or the right eye or an average of the axial length of the left eye and the axial length of the right eye and/or
- at least one corneal parameter of the left eye and/or the right eye and/or
- an indication of a presence of myopia on the left eye and/or the right eye and/or
- a variation of at least one among the spherical error, the monocular spherical equivalent, the binocular spherical equivalent, the refraction, the prescription, the axial length, the at least one corneal parameter or the indication, for a given period of time.

6. The computer-implemented method according to any one of the claims 1 to 5, wherein the behaviour of the user comprises proportions of time spent in near and far vision.

7. The computer-implemented method according to any one of the claims 1 to 6, wherein the sightedness impairment control solution (103) comprises at least one ophthalmic lens and the at least one characteristic of the sightedness impairment control solution is an optical characteristic.

8. The computer-implemented method according to claim 7 wherein the optical characteristics of the sightedness impairment control solution (103) comprise an optical power in at least one location of the at least one ophthalmic lens.

9. The computer-implemented method according to claim 8, wherein the at least one location comprises at least one of a near vision point or a far vision point of the at least one ophthalmic lens.

10. The computer-implemented method according to any one of the claims 1 to 6, wherein the sightedness impairment control solution (103) is a myopia control solution and wherein the myopia control solution comprises at least a device for implementing one of the following actions or several of the following actions simultaneously:
- correcting or reducing an accommodative lag during near vision activities;
- correcting peripheral hyperopic defocus or providing myopic defocus by;
- providing retinal stimulation;
- providing a different contrast in a peripheral vision of the user;
- limiting the amount of red light entering an eye of the user to reduce chromatism of the eye;
- providing light having a specific wavelength to the eye to inhibit eye lengthening;
- providing dynamically varying light stimuli such as flickering;
- providing drugs to regulate retinal and scleral muscarinic receptors;
- reshaping a cornea of the eye to reduce refraction defects;
- providing myopic defocus in the retinal periphery and/or optical aberrations by flattening the shape of the cornea,
wherein the at least one characteristic of the sightedness impairment control solution (103) being representative of the actions implemented by the device.

11. The computer-implemented method according to any one of the claims 1 to 10, also comprising:
- a step (501) of determining a first optical article based on the at least one first future characteristic of the user and,
- a step (502) of determining a second optical article based on the at least one second future characteristic of the user and
wherein the first image is an image of a face of the user wearing the first optical article and the second image is an image of the face of the user wearing the second optical article.

12. The computer-implemented method according to any one of the claims 1 to 10, also comprising:
- a step (501) of determining a first optical article based on the at least one first future characteristic of the user and,
- a step (502) of determining a second optical article based on the at least one second future characteristic of the user and
wherein the first optical article comprises a first lens and the second optical article comprises a second lens, the first image being an image of a shape of the first lens the second image being an image of a shape of the second lens.

13. An apparatus (102) for providing a user with a representation of an effect of a sightedness impairment control solution -103) on the user, the apparatus comprising:
a memory (102-a); and at least one processor (102-b) coupled to the memory (102-a) and configured to:
- obtain data representative of at least one characteristic of the sightedness impairment control solution, at least one current characteristic of the user and behaviour of the user,
- determine, based on a model and the data, at least one first future characteristic of the user will need if the user uses the sightedness impairment control solution (103) and at least one second future characteristic of the user will need if the user does not use the sightedness impairment control solution (103), and
- generate a first image based on the at least one first future characteristic and a second image based on the at least one second future characteristic.

14. A system (101) comprising, the apparatus (102) of claim 13 and a sightedness impairment control solution (103).

15. A computer program for providing a user with a representation of an effect of a sightedness impairment control solution (103) on the user, the computer program comprising instructions which, when the computer program is executed by a computer, cause the computer to carry out steps of:
- obtaining (301) data representative of at least one characteristic of the sightedness impairment control solution, at least one current characteristic of the user and behaviour of the user,
- determining (302), based on a model and the data, at least one first future characteristic of the user if the user uses the sightedness impairment control solution (103) and at least one second future characteristic of the user if the user does not use the sightedness impairment control solution (103), and
- generating (303) a first image based on the at least one first future characteristic and a second image based on the at least one second future characteristic.
